# EUROPEAN PATENT SPECIFICATION

(11) **EP 3 563 847 B1**
(45) Date of publication and mention of the grant of the patent: **20.09.2023**
(21) Application number: 17887220.6
(22) Date of filing: 28.12.2017
(51) Int. Cl.: A61K 9/20, A61K 9/50, A61K 9/16, A61K 31/397, A61K 9/00

(54) **PHARMACEUTICAL COMPOSITION**
PHARMAZEUTISCHE ZUSAMMENSETZUNG
COMPOSITION MÉDICINALE

(30) Priority: 28.12.2016 JP 2016255625
(43) Date of publication of application: 06.11.2019
(73) Proprietor: FUJIFILM Toyama Chemical Co., Ltd., Chuo-ku Tokyo 1040031 (JP)
(72) Inventor: OKADA, Kotaro, Ashigarakami-gun Kanagawa 258-8577 (JP); SAKATA, Yoshinori, Ashigarakami-gun Kanagawa 258-8577 (JP); TSUJIHATA, Shigetomo, Ashigarakami-gun Kanagawa 258-8577 (JP)
(74) Representative: Hoffmann Eitle
(86) International application number: PCT/JP2017/047254
(87) International publication number: WO 2018/124284

(56) References cited:
- EP-A1- 1 452 177
- EP-A1- 2 048 145
- EP-A1- 2 818 165
- EP-A1- 3 100 725
- WO-A1-2008/016107
- WO-A1-2013/125617
- WO-A1-2015/115582
- JP-A- 2006 519 202
- JP-A- 2009 263 298
- JP-A- 2010 208 987

## Description

### Technical Field

The present invention relates to a solid pharmaceutical composition comprising a predetermined drug and a compound having at least one hydrocarbon group having 8 to 18 carbon atoms and a sulfo group or a salt thereof.

### Background Art

1-(3-(2-(1-benzothiophen-5-yl)ethoxy)propyl)azetidin-3-ol (hereinafter also referred to as compound A) or a salt thereof has a neuroprotective action, a nerve regeneration promoting action, and a neurite outgrowth promoting action, and is a compound useful as a therapeutic agent for diseases of central and peripheral nerves (Patent Document 1).

Compound A or a salt thereof is orally administered. Heretofore, tablets comprising compound A or a salt thereof, lactose, crystalline cellulose, and excipients have been known (Patent Document 2).

Compound A has a characteristic bitterness. As a method of suppressing bitter taste, a method of adding a surfactant (Patent Document 3), a method of adding an organic acid (Patent Document 4), and a method of adding sodium lauryl sulfate (Patent Document 5) are known.

### Prior Art Documents

### Patent Documents

Patent Document 1: International Publication No. WO 2003/035647
Patent Document 2: International Publication No. WO 2013/125617 (family member of EP 2 818 165 A1)
Patent Document 3: JP Patent Publication (Kokai) No. 2010-208987
Patent Document 4: JP Patent Publication (Kokai) No. 2009-263298
Patent Document 5: JP Patent Publication (Kohyo) No. 2006-519202 (family member of EP 1 452 177 A1)

EP 3 100 725 A1 provides a medicinal composition comprising an alkyl ether derivative which is useful as a post nerve injury rehabilitation effect-enhancing agent.

EP 2 048 145 A1 discloses a protein kinase C enhancer containing a benzothiophene alkyl ether derivative. This protein kinase C enhancer is useful for treatment or prevention of various diseases associated with protein kinase C.

### Summary of Invention

### Object to be Solved by the Invention

Patients requiring compound A or a salt thereof are mainly elderly people, and an easier-to-take formulation is desired. Fine granules and orally disintegrating tablets are known as easy-to-take formulations, and a problem is that compound A has a characteristic bitterness.

The techniques disclosed in Patent Documents 3 and 4 are insufficient to suppress the characteristic bitterness of compound A or a salt thereof. Patent Document 5 shows only the effect of the specific compounds: epinastine and quinine on the bitter taste, and does not disclose the suppression of the post-bitter taste.

The object of the present invention is to provide a solid pharmaceutical composition in which both the pre-bitter taste and the post-bitter taste of compound A or a salt thereof are suppressed.

### Means for Solving the Object

The present inventors have intensively studied to solve the above problem, and have found that combining compound A or a salt thereof with a compound having at least one hydrocarbon group having 8 to 18 carbon atoms and a sulfo group or a salt thereof, which is selected from sodium lauryl sulfate and dioctyl sulfosuccinate sodium, allows the manufacture of a solid pharmaceutical composition with reduced bitter taste: both the pre-bitter taste and post-bitter taste, characteristic of compound A. Furthermore, the present inventors have found that a solid pharmaceutical composition can be manufactured, in which the bitter taste is improved by blending sodium lauryl sulfate or dioctyl sulfosuccinate sodium in fine granules comprising compound A or the salt thereof, and the dissolution properties in the digestive tract are maintained. The present invention has been completed based on these findings.

The present invention provides a solid pharmaceutical composition as defined in the claims.

### Advantageous Effects of Invention

The present invention provides a solid pharmaceutical composition in which both the pre-bitter taste and the post-bitter taste of compound A or a salt thereof are suppressed.

### Brief Description of Drawing

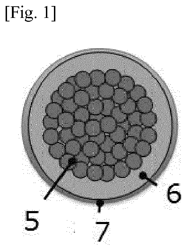

Fig. 1 shows a schematic view of an example of the fine granules of the present invention.

### Embodiment of Carrying out the Invention

In the present description, the numerical range shown using "to" means a range which includes the numerical value described before and after "to" as the minimum value and the maximum value, respectively.

In the present description, the amount of each component in the composition is, when a plurality of substances corresponding to the component are present in the composition, the total amount of the plurality of substances present in the composition, unless otherwise stated.

In the present description, "average particle size" means volume average particle size (Mv), which is a value measured using a laser diffraction scattering type particle size distribution measuring apparatus (product name: LS 13 320, Beckman Coulter, Inc.), and the method of measuring the average particle size is not particularly limited.

In the present description, the term "layer" includes not only the configuration of coating the entirety of the object to be coated and but also the configuration of coating a portion of the object to be coated.

### [Solid pharmaceutical composition]

The solid pharmaceutical composition of the present invention is a solid pharmaceutical composition as defined in the claims, comprising compound A or a salt thereof, and a compound having at least one hydrocarbon group having 8 to 18 carbon atoms and a sulfo group or a salt thereof, which is selected from sodium lauryl sulfate and dioctyl sulfosuccinate sodium.

In the present invention, the bitter taste of compound A or a salt thereof has been found to have not only a bitter taste felt when contained in the oral cavity (pre-bitter taste) and but also a bitter taste which persists after swallowing (post-bitter taste). That is, compound A or a salt thereof is a compound having both the pre-bitter taste and the post-bitter taste.

In the present invention, the following new effect has been found: by blending sodium lauryl sulfate or dioctyl sulfosuccinate sodium with compound A or a salt thereof, not only the pre-bitter taste felt in the oral cavity and but also the post-bitter taste that remains thereafter can be reduced. It has been found that by providing fine granules containing compound A or a salt thereof, a significant bitter taste masking effect can be obtained without affecting the dissolution properties of compound A or the salt thereof.

### <Compound A>

In the present invention, compound A (that is, 1-(3-(2-(1-benzothiophen-5-yl)ethoxy)propyl)azetidin-3-ol)) or a salt thereof is used as an active ingredient.

Since compound A has a cyclic amino group, examples of the salt thereof include salts at commonly known basic groups.

Examples of salts at basic groups include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylene sulfonic acid, and naphthalene sulfonic acid.

Among the above salts, preferable salts are pharmacologically acceptable salts and more preferable salts are salts with maleic acid.

In the case of having isomers (for example, optical isomers, geometric isomers, and tautomers), compound A or a salt thereof may be any of all these isomers and may be any of hydrates, solvates, and all crystal forms.

Compound A or a salt thereof can be manufactured by methods known per se or an appropriate combination thereof, or a method disclosed in Patent Document 1.

The content of compound A or the salt thereof in fine granules is 30 to 90% by mass, preferably 40 to 90% by mass, and more preferably 50 to 90% by mass.

### <Compound selected from sodium lauryl sulfate and dioctyl sulfosuccinate sodium >

The compound having a hydrocarbon group and a sulfo group or a salt thereof is selected from sodium lauryl sulfate and dioctyl sulfosuccinate sodium.

The structure of these compounds is shown below.

### Sodium lauryl sulfate

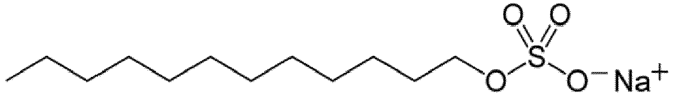

### Dioctyl sulfosuccinate sodium

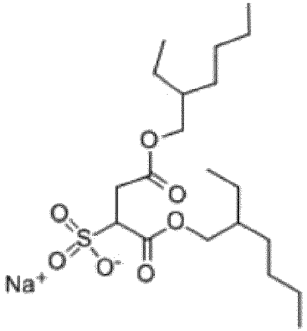

The amount of the compound having a hydrocarbon group and a sulfo group, which is selected from sodium lauryl sulfate and dioctyl sulfosuccinate sodium, is generally 1% by mass to 150% by mass, preferably 1% by mass to 100% by mass, and more preferably 1% by mass to 50% by mass, based on the mass of compound A or the salt thereof.

### <Fine granule>

The solid pharmaceutical composition of the present invention is a composition comprising fine granules comprising compound A or a salt thereof and a compound selected from sodium lauryl sulfate and dioctyl sulfosuccinate sodium.

The fine granules are fine granules having a core comprising compound A or a salt thereof and a binder, and a polymer layer with which the surface of the above core is coated.

The schematic diagram of an example of the above fine granules is shown in Fig. 1. In the fine granules shown in Fig. 1, the surface of a core 5 including the drug substance which is compound A is coated with a polymer layer 6, and the surface of the polymer layer 6 is further coated with an overcoat layer 7. The polymer layer 6 is a layer that functions as a bitter taste masking layer. The fine granules shown in Fig. 1 can increase the content of the drug substance included in one granule.

The core contains at least compound A or a salt thereof and the binder. The core preferably consists of core particles containing compound A or a salt thereof and the binder, and preferably does not contain core particles that do not contain compound A or a salt thereof. The method of preparing the core is not particularly limited, and a wet agitation method or the like is preferable. The method of preparing the core will be described below.

The surface of the core is coated with a polymer layer. The method of coating with a polymer layer is not specifically limited, and a method of spray-coating a core with the coating solution including the polymer is preferable. The method of forming the polymer layer will be described below.

The roundness of the fine granules is 0.8 or more, preferably 0.82 or more, more preferably 0.83 or more, further more preferably 0.84 or more, still more preferably 0.86 or more, and particularly preferably 0.87 or more. The upper limit of the roundness of fine granules is not particularly limited, and is at most 1.0.

The roundness is determined by microscopic observation of 5 to 15 particles and calculation of 4π×(area)/(square of perimeter) for the core using software (ImageJ, National Institutes of Health), and is expressed by the average value. In the case of a perfect circle, the roundness is 1.0.

### <Binder>

The type of the binder used to form the core is not particularly limited as long as it is a substance that can be mixed with compound A or a salt thereof to form the core. Examples of the binder include hydroxypropyl cellulose, hydroxypropyl methyl cellulose, methyl cellulose, carmellose sodium, gum arabic, pregelatinized starch, polyvinyl alcohol (PVA), and polyvinyl alcohol/polyethylene glycol graft polymer. The binders may be used singly or in combinations of two or more. Among them, the binder is preferably hydroxypropyl cellulose, gum arabic, pregelatinized starch, or PVA, and particularly preferably hydroxypropyl cellulose.

The amount of a binder used is not particularly limited, and is preferably 2 to 30% by mass, more preferably 3 to 30% by mass, further more preferably 3 to 20% by mass, still more preferably 5 to 20% by mass, and particularly preferably 5 to 15% by mass, based on the mass of compound A or the salt thereof.

### <Additive in core>

The core may consist of compound A or a salt thereof and the binder, or may be added with additives such as excipients, in addition to compound A or a salt thereof and the binder. Examples of additives can include sugars selected from crystalline cellulose, lactose monohydrate, sucrose, and glucose; sugar alcohol selected from mannitol, sorbitol, erythritol, maltitol, trehalose, xylitol, and isomalt; cellulose derivatives such as ethylcellulose, carmellose, carmellose calcium, croscarmellose sodium, and low substituted hydroxypropyl cellulose; starch derivatives such as sodium carboxymethyl starch and pregelatinized starch; polypyrrolidone derivatives such as crospovidone; cyclodextrins such as α-cyclodextrin, β-cyclodextrin, γ-cyclodextrin, hydroxypropyl β-cyclodextrin, and sulfobutylether β-cyclodextrin sodium; starches such as corn starch, potato starch, and partially pregelatinized starch; phosphates such as calcium hydrogen phosphate and anhydrous calcium hydrogen phosphate; and carbonates such as precipitated calcium carbonate, and lactose monohydrate is particularly preferable.

The content of additives such as excipients in the core is not particularly limited, and is generally 10 to 100% by mass based on the mass of compound A or the salt thereof.

### <Polymer layer>

The surface of the core can be coated with a polymer layer. The type of a polymer constituting the polymer layer is not particularly limited as long as it can be used in the pharmaceutical composition, that is, pharmaceutically acceptable, and for example, ammonio alkyl methacrylate copolymer (such as product name: Eudragit (R) RS 100, which is a pH-independent sustained release coating), methacrylic acid copolymer, ethyl cellulose, and ethyl acrylate/methyl methacrylate copolymer (such as product name: Eudragit (R) NE30D, which is a pH-independent sustained release coating) can be used. As the polymer, one may be used alone, or two or more polymers may be used in combination.

The amount of the polymer used is not particularly limited, and is preferably 5 to 100% by mass, more preferably 10 to 90% by mass, still more preferably 20 to 80% by mass based on the mass of compound A or the salt thereof.

The polymer layer may consist of the above described polymer or may comprise additives other than the above described polymer.

Examples of additives in the polymer layer include plasticizer (for example, triacetin and triethyl citrate), and surfactant (glyceryl monostearate, polysorbate 80 and the like), and are not particularly limited.

The amount of the above additive used is not particularly limited, and the content of each additive is generally 2 to 30% by mass based on the mass of the polymer.

### <Overcoat layer>

The fine granules may further have an overcoat layer with which the surface of the polymer layer is coated. The fine granules having the overcoat layer can further suppress adhesion or aggregation among fine granules.

The type of a component constituting the overcoat layer is not particularly limited as long as it can be used in the pharmaceutical composition, that is, pharmaceutically acceptable, and for example, excipients such as mannitol, anhydrous silicic acid (light anhydrous silicic acid and the like) can be used.

The content of the overcoat layer is generally 0.5% by mass to 20% by mass based on the total mass of the core, the polymer layer, and the overcoat layer.

### <Content of compound selected from sodium lauryl sulfate and dioctyl sulfosuccinate sodium >

The content of the compound selected from sodium lauryl sulfate and dioctyl sulfosuccinate sodium in the fine granules is generally 1% by mass to 50% by mass, preferably 1% by mass to 30% by mass, more preferably 2% by mass to 20% by mass, still more preferably 3% by mass to 15% by mass, based on the mass of compound A or a salt thereof.

### <Average particle size of fine granules>

The average particle size of fine granules in the present invention is 100 µm to 1000 µm, preferably 100 µm to 750 µm, and more preferably 100 µm to 500 µm. By setting the average particle size of the fine granules within the above range, the roughness in the oral cavity can be further reduced when the pharmaceutical composition is disintegrated in the oral cavity, and thus an unpleasant feeling of taking can be avoided.

### [Method of manufacturing solid pharmaceutical composition]

The solid pharmaceutical composition of the present invention can be manufactured by the manufacturing method comprising: a step of manufacturing the core comprising compound A or a salt thereof and the binder by the wet agitation granulation method using compound A or a salt thereof and the binder; and a step of forming the polymer layer on the surface of the above core.

### <Manufacturing of core comprising compound A or salt thereof and binder>

The core can be manufactured by the wet agitation granulation method using compound A or a salt thereof and the binder. The wet agitation granulation method is a method of manufacturing aggregated particles of powder by agitating while adding a suitable liquid binder to a fine powder.

The wet agitation granulation method can be performed using an agitated granulator. An example of an agitated granulator can include vertical granulator model FM-VG-01, manufactured by Powrex Corporation, and is not particularly limited.

The aqueous solution of the binder can be sprayed while agitating compound A or a salt thereof and the binder in the agitated granulator. Then, the agitation is further performed, and a granulated material can be obtained.

The agitation speed at spraying the aqueous solution of the binder is not particularly limited, and the blade rotational speed can be preferably 200 rpm to 400 rpm and more preferably 250 rpm to 300 rpm, and the cross screw rotational speed can be preferably 1000 rpm to 4000 rpm and more preferably 2000 rpm to 3000 rpm.

The agitation speed of the agitation after spraying the aqueous solution of the binder is not particularly limited, and the blade rotational speed can be preferably 600 rpm to 1000 rpm and more preferably 700 rpm to 900 rpm, and the cross screw rotational speed can be preferably 1000 rpm to 4000 rpm and more preferably 2000 rpm to 3000 rpm.

Drying the granulated material obtained above allows manufacturing of a core comprising compound A or a salt thereof and a binder. Drying can be performed using a fluidized bed granulator (for example, model FD-MP-01, manufactured by Powrex Corporation).

### <Step of forming polymer layer on surface of core>

The step of forming the polymer layer on the surface of the core can be performed by a general coating method. For example, a coating solution for a polymer layer may be prepared by dissolving the components constituting the polymer layer in a solvent and sprayed onto the core.

The compound selected from sodium lauryl sulfate and dioctyl sulfosuccinate sodium is preferably present in the outermost layer of fine granules.

When the polymer layer is the outermost layer of the fine granules, the polymer layer can comprise this compound.

The spray speed, spray time, solution temperature, drying conditions, and the like of the coating solution for a polymer layer are not particularly limited, and may be appropriately set according to the composition of the coating solution for a polymer layer, viscosity, particle size, and the like.

### <Step of forming overcoat layer on surface of polymer layer>

Forming the overcoat layer on the surface of the polymer layer can manufacture fine granules having the overcoat layer with which the surface of the polymer layer is coated. However, the overcoat layer may or may not be provided.

When an overcoat layer is provided and the overcoat layer is the outermost layer of fine granules, the overcoat layer can comprise a compound selected from sodium lauryl sulfate and dioctyl sulfosuccinate sodium.

The step of forming the overcoat layer can be performed by a general coating method and a powder addition method. For example, a coating solution for an overcoat layer may be prepared by dissolving the components constituting the overcoat layer in a solvent and sprayed onto the surface of the polymer layer. The spray speed, --+ --+ --+ Page 15 spray time, solution temperature, drying conditions, and the like of the coating solution for an overcoat layer are not particularly limited, and may be appropriately set according to the composition of the coating solution for an overcoat layer, viscosity, particle size, and the like. In the powder addition method, the components constituting the overcoat layer may be added in the form of a powder and mixed.

### [Form of solid pharmaceutical composition]

The form of the solid pharmaceutical composition of the present invention is not particularly limited, and is preferably a solid formulation for oral use such as fine granules, tablets, and granules, and more preferably fine granules or tablets. The tablet is preferably an orally disintegrating tablet.

The tablet can be conventional coated tablets, for example, sugar-coated tablets, gelatin-encapsulated tablets, gastric-coated tablets, enteric-coated tablets, and water-soluble film-coated tablets, as required.

The tablet can be preferably an orally disintegrating tablet.

The orally disintegrating tablet may further comprise an excipient component outside the fine granules. The excipient component as used herein is a component that can contribute to improvement in the formability and the ease of taking of the tablet comprising fine granules. The excipient component may comprise pharmacologically acceptable pharmaceutical additives such as bitter taste inhibitors, odor adsorbents, excipients, disintegrants, lubricants, binders, fluidizers, sweeteners, flavors, and coloring agents, in the range which does not inhibit the effect of the present invention. The pharmaceutical additive may be one in which one component performs two or more functions. Specific examples of the excipient component can include those described in paragraph Nos. 0085 to 0095 of JP Patent Publication (Kokai) No. 2016-141630.

As the pharmaceutical additive, one may be used alone, or two or more may be used in combination.

The content of pharmaceutical additives in the excipient component comprised outside of fine granules can be set appropriately in consideration of the content ratio of fine granules in the orally disintegrating tablet, the average particle size of the fine granules, and the like.

### [Properties of solid pharmaceutical composition]

When the solid pharmaceutical composition of the present invention is a tablet, 40 to 1000 mg of compound A or a salt thereof per tablet is preferably comprised.

The shape of the solid pharmaceutical composition of the present invention is not particularly limited as long as it is pharmaceutically acceptable.

When the solid pharmaceutical composition of the present invention is a tablet, the shape of the tablet may be, for example, a round tablet or a modified tablet, and can be appropriately set in consideration of drug compliance.

When the solid pharmaceutical composition of the present invention is a tablet, the size of the tablet is not particularly limited as long as it is pharmaceutically acceptable. From the view point that tablets are often used for patients who have difficulty swallowing, the size of the tablet of the present invention is preferably as small as possible in consideration of its medicinal effects.

In the solid pharmaceutical composition of the present invention, from the viewpoint of drug efficacy, dissolution rate after 60 minutes in the Japanese Pharmacopoeia dissolution test (paddle method: paddle rotation speed of 50 rpm, dissolution test solution: 0.1 mol/L hydrochloric acid) assuming dissolution of drug in the stomach is preferably 40% or more, more preferably 45% or more, further more preferably 50% or more, still more preferably 60% or more, yet still more preferably 70% or more, and particularly preferably 80% or more.

### [Method of manufacturing tablets]

When the solid pharmaceutical composition of the present invention is a tablet, the method of manufacturing the tablet is not particularly limited, and known methods can be used. The tablet of the present invention can be obtained, for example, by mixing fine granules and, if desired, an excipient component, tableting the obtained mixed powder, and drying it.

The method of mixing the fine granules and the excipient component is not particularly limited. Examples of the method of mixing the fine granules and the excipient component include a method of mixing using a known mixer such as a V-type mixer (Tsutsui Scientific Instruments Co., Ltd.) and a fluidized bed granulator (Powrex Corporation).

The conditions such as the time required for mixing can be appropriately adjusted depending on the types of fine granules and the excipient component.

The method of tableting the mixed powder of fine granules and an excipient component is not particularly limited. The temperature at tableting is not particularly limited, and can be appropriately set.

An example of the method of tableting the mixed powder of fine granules and an excipient component includes a method of tableting using a tableting machine such as a rotary tableting machine (product name: HT-AP-SS, Hata Tekkosho Co., Ltd.) or a high-speed rotary type tableting machine (product name: AQUARIUS G, Kikusui Seisakusho Ltd.).

Method of drying tableted material obtained by tableting the mixed powder is not particularly limited. Examples of the method of drying tableted material obtained by tableting the mixed powder include a method of drying by vacuum drying, fluidized bed drying, or the like.

### [Administration of solid pharmaceutical composition]

The administration method, dosage, and frequency of administration of the solid pharmaceutical composition of the present invention can be appropriately selected according to the age, body weight, and symptoms of the patient. The dose that can exert its medicinal effects may be typically administrated in a single dose or in several divided doses a day, and the dose of compound A or a salt thereof, for example, 40 to 1000 mg may typically be administrated to an adult in a single dose or in several divided doses a day.

Hereinafter, the present invention will be described in detail by the following Examples, but the present invention is not limited to these Examples.

### Examples

### Reference Examples 1 and 2 and Comparative Examples 1 to 7

The following evaluation was performed about a solid pharmaceutical compositions obtained by mixing the maleate of compound A and an additive according to the compositions shown in Table 1 and Table 2. The results of the above evaluation are shown in Table 1 and Table 2.

### <Evaluation of bitter taste (pre-bitter taste and post-bitter taste)>

Evaluation was performed using a taste sensing system (Intelligent Sensor Technology, Inc.: SA402B). This taste sensing system is a device capable of taking out a change in the membrane potential of the artificial lipid membrane as a signal, and a relative value corresponding to the pre-taste and a CPA value corresponding to the aftertaste can be obtained. The relative value and the CPA value are defined as follows:
Relative value: Vs-Vr
CPA value: Vr'-Vr. Change of membrane Potential caused by Adsorption.
Vr (mV): Measured value of reference solution before measuring control solution or sample solution
Vs (mV): Measured value of the control solution or the sample solution
Vr'(mV): Measured value of the reference solution measured again after measuring the control solution or sample solution

The measurement was performed three times, and the average value was calculated for each relative value and each CPA value of the control solution and each sample solution.

In the taste sensing system, after stabilization with reference solutions (30 mmol/L KCl and 0.3 mmol/L tartaric acid), relative values and CPA values in the control solution and each sample solution were measured. As the sensor used for measurement, AC0 specific to basic bitter taste was used.

Separately, a sensory test was performed, and a calibration curve was created from the correlation between the bitter taste score of the sensory, and the relative value and the CPA value. The bitter taste score was calculated by interpolating the relative value and the CPA value from the calibration curve. The pre-bitter taste score was calculated from the relative value and the post-bitter taste score was calculated from the CPA value.

### Bitter taste score of sensory:

1 Bitter taste that can be perceived at last
2 Understandable bitter taste
3 Bitter taste that can be perceived easily
4 Strong bitter taste
5 Unbearable bitter taste

The test method is as follows.

The fine granules were added to a 10 mmol/L KCl solution such that the concentration of the maleate of compound A was 1 mg/mL, and the mixture was agitated for 30 minutes with a stirrer to give a sample solution. In addition, a 10 mmol/L KCl solution was used as a control solution.

The evaluation criteria for bitter taste (pre-bitter taste and post-bitter taste) are as follows:
A Less than 0.5
B 0.5 or more and less than 1.5
C 1.5 or more and less than 2.5
D 2.5 or more

### [Table 1]

**Table 1: A numerical value in the table shows parts by mass**

| | | Example 1 | Example 2 | Comparative Example 1 | Comparative Example 2 |
|---|---|---|---|---|---|
| Maleate of compound A | | 1 | 1 | 1 | 1 |
| Additive | Sodium lauryl sulfate | 1 | | | |
| | Dioctyl sulfosuccinate sodium | | 1 | | |
| | PEG6000 | | | 1 | |
| | Polyoxyethylene cured castor oil | | | | 1 |
| Bitter taste | Pre-bitter taste | A | B | D | D |
| | Post-bitter taste | C | B | C | C |

| | | | | | |
|---|---|---|---|---|---|
| Examples 1 and 2 are Reference Examples. | | | | | |

### [Table 2]

**Table 2: A numerical value in the table shows parts by mass**

| | | Comparative Example 3 | Comparative Example 4 | Comparative Example 5 | Comparative Example 6 | Comparative Example 7 |
|---|---|---|---|---|---|---|
| Maleate of compound A | | 1 | 1 | 1 | 1 | 1 |
| Additive | Polysorbate 80 | 1 | | | | |
| | Taurine | | 1 | | | |
| | Chondroitin sodium sulfate | | | 1 | | |
| | Oleic acid | | | | | 1 |
| Bitter taste | Pre-bitter taste | D | D | D | D | D |
| | Post-bitter taste | C | D | D | D | D |

### Examples 3 and 4 (wet agitation)

### (Formation of core)

268.8 g of the maleate of compound A and 25.2 g of hydroxypropyl cellulose (HPC-L: product name, Nippon Soda Co., Ltd.) were placed in an agitation granulator (vertical granulator model FM-VG-01, manufactured by Powrex Corporation) and sprayed with 60.0 g of 10% by mass of hydroxypropyl cellulose (HPC-L: product name, Nippon Soda Co., Ltd.) aqueous solution while agitated at a blade rotation speed of 270 rpm and a cross screw rotation speed of 2500 rpm. Then, agitation was performed for 9 minutes at a blade rotation speed of 800 rpm and a cross screw rotation speed of 2500 rpm. The whole amount of the obtained granulated material was dried using a fluidized bed granulator (model FD-MP-01, manufactured by Powrex Corporation) at an air supply temperature of 60°C to obtain a core.

### (Formation of polymer layer)

Among the obtained cores, a core having a particle size of 100 µm to 355 µm was recovered using a sieve; 10 g of the core was placed in a micro fluidized-bed apparatus (manufactured by Dalton) which was a fluidized bed granulator; the air supply temperature was set to room temperature (30 to 40°C); a coating solution containing ammonioalkyl methacrylate copolymer dispersion (Eudragit RS30D: product name, Evonik), triethyl citrate (Citroflex 2: product name, Morimura Corporation), glyceryl monostearate (Riken Vitamin), polysorbate 80 (NOF Corporation), and purified water was fed into the micro fluidized-bed apparatus at a rate of 0.2 to 0.3 g/minute to perform spray coating; and thereby a polymer layer was formed.

### (Formation of overcoat layer)

After forming the polymer layer, a solution containing mannitol (the content of mannitol is 14%) was fed into the micro fluidized-bed apparatus at 0.3 g/minute to obtain particles comprising an overcoat layer on the surface of the polymer layer. A coating solution containing sodium lauryl sulfate (the content of sodium lauryl sulfate is 5%) is fed into the micro fluidized-bed apparatus at a rate of 0.3 g/minute to obtain particles comprising sodium lauryl sulfate in the above obtained particles. Among the obtained particles, particles having a particle size of 100 µm to 425 µm were recovered using a sieve and used for various evaluations.

### Example 5 (Wet agitation)

A core was formed in the same manner as in Example 3.

The formation of the polymer layer was performed in the same manner as in Example 3 except that a coating solution containing ethylcellulose, ethyl acrylate/methyl methacrylate copolymer, triacetin, and purified water was used as a coating solution.

The formation of the overcoat layer was performed in the same manner as in Example 3.

### Comparative Example 8 (Wet agitation)

The formation of the core and the formation of the polymer layer were performed in the same manner as in Example 3.

The formation of the overcoat layer was performed in the same manner as in Example 3 except that a coating solution containing mannitol and purified water (sodium lauryl sulfate was not contained) was used.

### [Evaluation]

The following items were evaluated for the pharmaceutical compositions manufactured in Examples and Comparative Examples. The results of the evaluation are shown in Table 3.

### <Evaluation of bitter taste>

Evaluation was performed in the same manner as described in Reference Examples 1 and 2 and Comparative Examples 1 to 7.

However, the measurement method is as follows. The pharmaceutical composition was added to Japanese Pharmacopoeia purified water such that the concentration of the maleate of compound A was 9 mg/mL, and mixed by inversion for 30 seconds. The solution was immediately filtered through a polysilicate glass fiber, and the solution to which KCl was added such that the KCl concentration in the filtrate was 10 mmol/L was used as a sample solution. In addition, a 10 mmol/L KCl solution was used as a control solution.

The criteria for evaluation are as follows.
A Less than 0.5
B 0.6 or more and less than 1.5
C 1.5 or more and less than 2.5
D 2.5 or more

### <Dissolution>

A dissolution test was performed in accordance with the dissolution test described in 16th revised Japanese Pharmacopoeia. The dissolution test was performed by a paddle method (paddle rotation speed: 50 rpm) using a dissolution test machine DT-810 of JASCO Corporation. As a dissolution test solution, 0.1 mol/L hydrochloric acid (volume: 900 mL) was used and degassed by a Media Prep of Nihon Validation Technologies Corporation; and then, the temperature therefor was maintained at 37 ± 0.5°C. The amounts of compound A released from a sample for a testing period of 30 minutes were measured using an ultraviolet spectrophotometer at wavelengths of 257 nm and 500 nm, and a dissolution rate (%) from a difference therebetween was calculated.

### [Table 3]

**Table 3: A numerical value of each component in the table shows parts by mass**

| | | Comparative Example 8 | Example 3 | Example 4 | Example 5 |
|---|---|---|---|---|---|
| Core particle | Maleate of compound A | 448 | 448 | 448 | 448 |
| | Hydroxypropyl cellulose | 52 | 52 | 52 | 25 |
| Polymer layer | Ethyl cellulose | | | | 95 |
| | Ethyl acrylate/methyl methacrylate copolymer | | | | 28 |
| | Triacetin | | | | 14 |
| | Ammonioalkyl methacrylate copolymer | 203 | 203 | 203 | |
| | Triethyl citrate | 10 | 10 | 10 | |
| | Glyceryl monostearate | 10 | 10 | 10 | |
| | Polysorbate 80 | 4 | 4 | 4 | |
| Overcoat layer | Mannitol | 89 | 89 | 89 | 78 |
| | Sodium lauryl sulfate (% relative to maleate of compound A) | 0 (0%) | 19 (-4%) | 57 (-13%) | 58 (-13%) |
| Bitter taste | Pre-bitter taste | C | A | A | A |
| | Post-bitter taste | B | B | A | A |
| Dissolution properties | Dissolution rate after 30 minutes of 0.1 mol/L hydrochloric acid | 92% | 93% | 94% | 96% |

### Reference Example 6

### (Formation of core)

The formation of the core and the formation of the polymer layer were performed in the same manner as in Example 3.

The formation of the overcoat layer was performed in the same manner as in Example 3 except that a coating solution containing mannitol and purified water (sodium lauryl sulfate was not contained) was used.

The fine granules obtained above and the excipient component were mixed in such a way as to provide the composition shown in Table 4, to obtain a tableted powder (mixed powder). The tableted powder (mixed powder) obtained was weighed out such that 448 mg of the maleate of compound A was contained in 3 tablets and was compression molded using a rotary tableting machine (product name: HT-AP-SS, Hata Tekkosho Co., Ltd.) with a 12 mmϕ single R face punch at a rotational speed of 20 rpm in such a way as to provide a hardness of 50 N, to obtain an orally disintegrating tablet (tablet).

**[Table 4]**

| | | | | Example 6 |
|---|---|---|---|---|
| Fine granule | Core | Maleate of compound A | 448 | 49.3 |
| | | Hydroxypropyl cellulose | 25 | |
| | Polymer layer | Ethyl cellulose | 149 | |
| | | Ethyl acrylate/methyl methacrylate copolymer | 45 | |
| | | Triacetin | 22 | |
| | Overcoat layer | Mannitol | 84 | |
| Granulated material of mannitol/corn starch | | | | 17.8 |
| Crospovidone | | | | 3.0 |
| Ethyl cellulose | | | | 3.9 |
| Mg aluminometasilicate | | | | 2.5 |
| Crystalline cellulose | | | | 19.7 |
| Hydrous silicon dioxide | | | | 1.0 |
| Aspartame | | | | 1.0 |
| Strawberry micron | | | | 0.1 |
| Ca stearate | | | | 0.3 |
| Na Lauryl sulfate | | | | 1.4 |

### [Evaluation of orally disintegrating tablet]

### <Evaluation of bitter taste masking>

One orally disintegrating tablet was placed in the oral cavity of the subject. Evaluation was performed according to the following evaluation criteria whether bitter taste derived from compound A remained 60 minutes after the placement.

### Evaluation criteria:

B A bitter taste was slightly felt 60 minutes after the placement
A No bitter taste was felt 60 minutes after the placement

The orally disintegrating tablet obtained in Reference Example 6 was subjected to sensory evaluation of bitter taste masking. As a result, for the orally disintegrating tablet in Reference Example 6, no bitter taste was felt, and the bitter taste derived from compound A was sufficiently masked.

### <Oral disintegration time>

One orally disintegrating tablet was dosed in the oral cavity of one adult male, and the time until the core of the tablet was not felt was measured.

The oral disintegration time of the orally disintegrating tablet in Reference Example 6 was measured. As a result, the oral disintegration time of Reference Example 6 was 41 seconds.

### Reference Signs List

- 5: Core
- 6: Polymer layer
- 7: Overcoat layer

## Claims

1. A solid pharmaceutical composition comprising fine granules having a core and a polymer layer with which a surface of the core is coated,
wherein the core comprises 1-(3-(2-(1-benzothiophen-5-yl)ethoxy)propyl)azetidin-3-ol or a salt thereof and a binder,
wherein the fine granules comprise a compound having at least one hydrocarbon group having 8 to 18 carbon atoms and a sulfo group or a salt thereof,
wherein the average particle size of the fine granules is 100 µm to 1000 µm, and wherein the compound having at least one hydrocarbon group having 8 to 18 carbon atoms and a sulfo group or the salt thereof is sodium lauryl sulfate or dioctyl sulfosuccinate sodium.

2. The solid pharmaceutical composition according to claim 1, wherein a content of the compound having at least one hydrocarbon group having 8 to 18 carbon atoms and a sulfo group or the salt thereof is 1 to 100% by mass based on the mass of 1-(3-(2-(1-benzothiophen-5-yl)ethoxy)propyl)azetidin-3-ol or the salt thereof.

3. The solid pharmaceutical composition according to claim 1 or 2, wherein the compound having at least one hydrocarbon group having 8 to 18 carbon atoms and a sulfo group or the salt thereof is sodium lauryl sulfate.

4. The solid pharmaceutical composition according to claim 1, 2 or 3, wherein the polymer layer comprises at least one selected from the group consisting of ammonioalkyl methacrylate copolymer, methacrylic acid copolymer, ethyl cellulose, and ethyl acrylate/methyl methacrylate copolymer.

5. The solid pharmaceutical composition according to claim 1, 2, 3 or 4, further comprising an overcoat layer with which a surface of the polymer layer is coated.

6. The solid pharmaceutical composition according to any one of claims 1 to 5, wherein the compound having at least one hydrocarbon group having 8 to 18 carbon atoms and a sulfo group or the salt thereof is present in an outermost layer of the fine granules.

7. The solid pharmaceutical composition according to any one of claims 1 to 6, wherein a content of the compound having at least one hydrocarbon group having 8 to 18 carbon atoms and a sulfo group or the salt thereof is 1 to 50% by mass based on the mass of 1-(3-(2-(1-benzothiophen-5-yl)ethoxy)propyl)azetidin-3-ol or the salt thereof.

8. The solid pharmaceutical composition according to any one of claims 1 to 7, wherein the composition is a fine granule or a tablet.

9. The solid pharmaceutical composition according to any one of claims 1 to 7, wherein the composition is an orally disintegrating tablet.

## Patentansprüche

1. Feste pharmazeutische Zusammensetzung umfassend feine Körnchen mit einem Kern und einer Polymerschicht, mit der eine Oberfläche des Kerns beschichtet ist,
wobei der Kern 1-(3-(2-(1-Benzothiophen-5-yl)ethoxy)propyl)azetidin-3-ol oder ein Salz davon und ein Bindemittel umfasst,
wobei die feinen Körnchen umfassen eine Verbindung mit mindestens einer Kohlenwasserstoffgruppe mit 8 bis 18 Kohlenstoffatomen und einer Sulfogruppe oder ein Salz davon,
wobei die durchschnittliche Teilchengröße der feinen Körnchen 100 µm bis 1000 µm beträgt, und wobei die Verbindung mit mindestens einer Kohlenwasserstoffgruppe mit 8 bis 18 Kohlenstoffatomen und einer Sulfogruppe oder das Salz davon, Natriumlaurylsulfat oder Dioctylsulfosuccinat-Natrium ist.

2. Feste pharmazeutische Zusammensetzung gemäß Anspruch 1, wobei der Gehalt der Verbindung mit mindestens einer Kohlenwasserstoffgruppe mit 8 bis 18 Kohlenstoffatomen und einer Sulfogruppe oder das Salz davon, 1 bis 100 Massen-% beträgt, bezogen auf die Masse von 1-(3-(2-(1-Benzothiophen-5-yl)ethoxy)propyl)azetidin-3-ol oder das Salz davon.

3. Feste pharmazeutische Zusammensetzung gemäß Anspruch 1 oder 2, wobei die Verbindung mit mindestens einer Kohlenwasserstoffgruppe mit 8 bis 18 Kohlenstoffatomen und einer Sulfogruppe oder das Salz davon, Natriumlaurylsulfat ist.

4. Feste pharmazeutische Zusammensetzung gemäß Anspruch 1, 2, oder 3, wobei die Polymerschicht umfasst mindestens eines, ausgewählt aus der Gruppe, bestehend aus Ammonioalkylmethacrylat-Copolymer, Methacrylsäure-Copolymer, Ethylcellulose und Ethylacrylat/Methylmethacrylat-Copolymer.

5. Feste pharmazeutische Zusammensetzung gemäß Anspruch 1, 2, 3, oder 4, ferner umfassend eine Überzugsschicht mit der eine Oberfläche der Polymerschicht beschichtet ist.

6. Feste pharmazeutische Zusammensetzung gemäß mindestens einem der Ansprüche 1 bis 5, wobei die Verbindung mit mindestens einer Kohlenwasserstoffgruppe mit 8 bis 18 Kohlenstoffatomen und einer Sulfogruppe oder das Salz davon, in einer äußersten Schicht der feinen Körnchen vorhanden ist.

7. Feste pharmazeutische Zusammensetzung gemäß mindestens einem der Ansprüche 1 bis 6, wobei der Gehalt der Verbindung mit mindestens einer Kohlenwasserstoffgruppe mit 8 bis 18 Kohlenstoffatomen und einer Sulfogruppe oder das Salz davon, 1 bis 50 Massen-% beträgt, bezogen auf die Masse von 1-(3-(2-(1-Benzothiophen-5-yl)ethoxy)propyl)azetidin-3-ol oder das Salz davon.

8. Feste pharmazeutische Zusammensetzung gemäß mindestens einem der Ansprüche 1 bis 7, wobei die Zusammensetzung ein feines Granulat oder eine Tablette ist.

9. Feste pharmazeutische Zusammensetzung gemäß mindestens einem der Ansprüche 1 bis 7, wobei die Zusammensetzung eine oral zerfallende Tablette ist.

## Revendications

1. Composition pharmaceutique solide comprenant des granulés fins ayant un noyau et une couche de polymère avec laquelle une surface du noyau est enrobée, dans laquelle le noyau comprend le 1-(3-(2-(1-benzothiophèn-5-yl)éthoxy)propyl) azétidin-3-ol ou un sel de celui-ci et un liant,
dans laquelle les granulés fins comprennent un composé ayant au moins un groupe hydrocarboné contenant 8 à 18 atomes de carbone et un groupe sulfo ou un sel de celui-ci,
dans laquelle la taille moyenne de particule des granulés fins est de 100 µm à 1 000 µm, et
dans laquelle le composé ayant au moins un groupe hydrocarboné contenant 8 à 18 atomes de carbone et un groupe sulfo ou le sel de celui-ci est le laurylsulfate de sodium ou le dioctylsulfosuccinate de sodium.

2. Composition pharmaceutique solide selon la revendication 1, dans laquelle une teneur en composé ayant au moins un groupe hydrocarboné contenant 8 à 18 atomes de carbone et un groupe sulfo ou le sel de celui-ci est de 1 à 100 % en masse sur la base de la masse du 1-(3-(2-(1-benzothiophèn-5-yl)éthoxy)propyl)azétidin-3-ol ou du sel de celui-ci.

3. Composition pharmaceutique solide selon la revendication 1 ou la revendication 2, dans laquelle le composé ayant au moins un groupe hydrocarboné contenant 8 à 18 atomes de carbone et un groupe sulfo ou le sel de celui-ci est le laurylsulfate de sodium.

4. Composition pharmaceutique solide selon la revendication 1, 2 ou 3, dans laquelle la couche de polymère comprend au moins un élément sélectionné dans le groupe consistant en un copolymère de méthacrylate d'ammonioalkyle, un copolymère d'acide méthacrylique, une éthylcellulose, et un copolymère d'acrylate d'éthyle/ méthacrylate de méthyle.

5. Composition pharmaceutique solide selon la revendication 1, 2, 3 ou 4, comprenant en outre une couche supérieure avec laquelle une surface de la couche de polymère est enrobée.

6. Composition pharmaceutique solide selon l'une quelconque des revendications 1 à 5,
dans laquelle le composé ayant au moins un groupe hydrocarboné contenant 8 à 18 atomes de carbone et un groupe sulfo ou le sel de celui-ci est présent dans une couche la plus externe des granulés fins.

7. Composition pharmaceutique solide selon l'une quelconque des revendications 1 à 6,
dans laquelle une teneur en composé ayant au moins un groupe hydrocarboné contenant 8 à 18 atomes de carbone et un groupe sulfo ou le sel de celui-ci est de 1 à 50 % en masse sur la base de la masse du 1-(3-(2-(1-benzothiophèn-5-yl)éthoxy) propyl)azétidin-3-ol ou du sel de celui-ci.

8. Composition pharmaceutique solide selon l'une quelconque des revendications 1 à 7, dans laquelle la composition est un granulé fin ou un comprimé.

9. Composition pharmaceutique solide selon l'une quelconque des revendications 1 à 7, dans laquelle la composition est un comprimé à désintégration orale.
